# EUROPEAN PATENT APPLICATION

(11) **EP 4 736 802 A1**
(43) Date of publication of application: **06.05.2026**
(21) Application number: 24210990.8
(22) Date of filing: 05.11.2024
(51) Int. Cl.: A61B 46/20, A61B 46/23

(54) **SURGICAL DRAPE WITH TRANSPARENT BARRIER SHEET**

(71) Applicant: Mölnlycke Health Care AB, 431 21 Mölndal (SE)
(72) Inventor: HERMOND, Johanna, 423 53 Torslanda (SE); JÖGÅRD WASS, Hanna, 432 37 Varberg (SE); SMID, Gerdie, 5253 AE Nieuwkuijk (NL); PAPE, Cheryl, 5051 ZV Goirle (NL)
(74) Representative: Kransell & Wennborg KB

(57) **Abstract**

The present disclosure relates to a surgical drape (1) having a lateral extension (x) and a longitudinal extension (y), the surgical drape being divided in an upper region (2) for arrangement proximal a head of a patient during surgical procedure and a lower region (3) arranged proximal a surgical site of the patient, the upper region being divided in a first lateral section (2a), a central section (2b) and a second lateral section (2c), the surgical drape further comprising:
- a surgeon facing barrier sheet (4) being essentially liquid impermeable and transparent, the surgeon facing barrier sheet extending over the first lateral section, the central section and the second lateral section of the upper region,
- a patient facing sheet (5) being essentially opaque, the patent facing sheet extending over the central section of the upper region,
- a fenestration (6) arranged in the lower region, the fenestration permitting access to the surgical site when the surgical drape covers a patient during surgical procedures,
- a first and a second limb portion (7,8) arranged in the surgeon facing barrier sheet in the upper region of the surgical drape,
wherein the surgeon facing barrier sheet extends beyond the patient facing sheet along a respective first and second longitudinal edge of the patient facing sheet in the upper region.

## Description

### TECHNICAL FIELD

The present disclosure relates to the field of surgical drapes and in particular to caesarean section (C-section) surgical drapes.

### BACKGROUND

In traditional caesarean section procedures, commonly known to as "C-sections," the patient is typically covered with a solid surgical drape. This drape is typically made of a multi-layered combination of spunbond and meltblown nonwoven materials, along with a liquid-impervious plastic film functioning as a barrier layer. The spunbond and meltblown nonwoven materials are absorbent materials which are also conventionally coloured materials.

During the procedure the mother is typically awake. Many conventional drapes are designed in a "T" shape, with the top portion of the "T" functioning as an anesthesia screen to provide a barrier or screen to create a sterile field around the abdomen. The anesthesia screen is held up on vertical supports at each side of the operating table near the patient's head or on a crossbar positioned close to the head.

In this setup, the mother may lack the opportunity to see her newborn immediately after delivery. However, establishing an immediate connection between mother and child in the first moments after birth is highly beneficial, particularly through skin-to-skin contact. Such contact is believed to offer numerous advantages for both mother and infant. Traditional surgical drapes, however, block the mother's view of her newborn in those initial moments, preventing this immediate physical connection. To address this limitation, some surgical drapes have been developed with a window and a coverable layer that may obscure or expose the window.

### SUMMARY

One objective of the present disclosure is to make available a surgical drape which enables the mother, during a C-section, to see and hold her newborn baby immediately after delivery, which surgical drape is also easy and convenient to use, both before and throughout the procedure, facilitating an enhanced bonding experience without compromising the sterile environment.

According to a first aspect, the present disclosure makes available a surgical drape having a lateral extension and a longitudinal extension, the surgical drape being divided in an upper region for arrangement proximal a head of a patient during surgical procedure and a lower region arranged proximal a surgical site of the patient, the upper region being divided in a first lateral section, a central section and a second lateral section, the surgical drape further comprising:
- a surgeon facing barrier sheet being essentially liquid impermeable and transparent, the surgeon facing barrier sheet extending over the first lateral section, the central section and the second lateral section of the upper region,
- a patient facing sheet being essentially opaque, the patent facing sheet extending over the central section of the upper region,
- a fenestration arranged in the lower region, the fenestration permitting access to the surgical site when the surgical drape covers a patient during surgical procedures,
- a first and a second limb portion arranged in the surgeon facing barrier sheet in the upper region of the surgical drape, the first and second limb portions each comprising a base portion attached to the surgical drape, a closed end portion and a sleeve portion extending between the base portion and the end portion,
wherein the surgeon facing barrier sheet extends beyond the patient facing sheet along a respective first and second longitudinal edge of the patient facing sheet in the upper region.

The term "essentially opaque" is used to describe the property where very little or no light passes through the material making object behind the material difficult or impossible to discern. Examples of such materials are nonwoven materials, such as multi-layer nonwovens, for examples combination of spunbond and meltblown nonwoven layer. The nonwoven material, or multilayer nonwoven may have a basis weight over 30 gsm, or over 40 gsm. A further example is a coloured nonwoven material. A further example is an opaque plastic film, such as a polyethylene film.

The patient facing sheet may have an opacity of at least 60%, or at least 70%, or at least 75%, or at least 80%, as measured according to the method ASTM D 2244-22.

The surgeon facing barrier sheet is a transparent, essentially liquid impermeable, sheet, such as a clear plastic film. The transparent barrier sheet may have a Haze level from 30% or lower, as measured according to ASTM D 1003. Optionally, the transparent barrier sheet has Haze level from 25% or lower, or 20% or lower, or 10% or lower, or 5% or lower, as measured according to ASTM D 1003. Optionally, the surgeon facing barrier sheet has a liquid penetration of ≥ 100 cm H₂O, as measured according to ISO 811.

The surgeon facing barrier sheet serves both as a protective barrier and a viewing window, allowing the mother to see her newborn baby immediately after delivery during a C-section. However, there may be instances during the procedure when it is necessary to obscure view, a function provided by the patient facing sheet.

The surgical drape is provided with limb portions, such as configured to receive the hands and arms of the patient, to provide designated openings where the mother can comfortably place her arms and hands to hold the baby immediately after delivery. This feature facilitates immediate skin-to-skin contact and bonding, allowing the mother to securely hold and connect with her newborn while maintaining a sterile environment.

The fact that the first and a second limb portion are arranged in the surgeon facing barrier sheet with the base portion attached to the surgical drape, compared to mittens attached to the patients' arms, allows for rapid removal of the patients, i.e. mothers, arms in case of an emergency.

Furthermore, when the surgical drape is positioned over the patient prior to the proceedings, the surgeon facing barrier sheet extends beyond the patient facing sheet on a respective first and second longitudinal edge in the upper region. This design of enables the patient to easily insert their arms into the first and second limb portions from a respective lateral side of the patient facing sheet without requiring the patient facing sheet to be removed.

The patient facing sheet is generally unattached to the surgeon facing barrier sheet in the upper region, or releasably attached. The patient facing sheet may alternatively be attached to the surgeon facing barrier sheet only proximal to the lower region or at least below the first and second limb portions, as seen in the longitudinal direction. Hence, the patient facing sheet may at least be unattached to the surgeon facing barrier sheet from the limb portions and above, as seen in the longitudinal direction.

The upper portion of the surgical drape, proximal a head of a patient during a procedure, may function as an anaesthesia screen establishing a sterile field around the abdomen during a C-section. The lower portion of the drape, designed to cover the abdomen and the lower part of the patient body, may comprise an absorbent sheet to manage fluids.

The fact that the surgeon facing barrier sheet extends beyond the patient facing sheet on a respective first and second longitudinal edge of the patient facing sheet enables the patient to insert the arms in the respective first and second limb portions in the surgeon facing barrier sheet without first removing the patient facing sheet.

Optionally, the patient facing sheet is an absorbent sheet, such as a liquid absorbent sheet. The patient facing sheet may be a nonwoven sheet.

Optionally, the patient facing sheet is a main sheet further extending over the lower region of the surgical drape. The patent facing sheet may form part of the upper part of the surgical drape and extend to the lower longitudinal edge of the surgical drape in the lower region. Such design provides a surgical drape which requires fewer components and still provides all the functions desired from the surgical drape.

Optionally, a width of the patient facing sheet in the lower region is greater than a width of the patient facing sheet in the upper region, preferably the width of the patient facing sheet is at least 30% greater in the lower region than the width in the upper region, optionally the width of the patient facing sheet is within the range of from 30% to 75% greater in the lower region than width in the upper region.

Optionally, the surgeon facing barrier sheet may have a width in the upper region, as measured in the lateral direction, within the range of from 350 cm and 200 cm.

Optionally, the patient facing sheet may have a width in the upper region, as measured in the lateral direction, within the range of from 75 cm and 150 cm.

Optionally, the surgeon facing barrier sheet extends beyond the patient facing sheet on a respective first and second longitudinal edge of the patient facing sheet with 30 cm or more per side, optionally 40 cm or more per side, preferably 50 cm or more per side. Such design further facilitates insertion of the patient's arms into the designated first and second limb portions prior to removing the patient facing sheet.

Optionally, a width of the patient facing sheet is greater in the lower region than in the upper region of the surgical drape. Preferably, the width of the patient facing sheet is at least 30% greater in the lower region than in the upper region, optionally the width of the patient facing sheet is within the range of from 30% to 75% greater in the lower region than in the upper region. The patient facing sheet arranged in the lower region is generally an absorbent sheet, such as a hydrophilic nonwoven sheet, and is suitable for absorbing fluids generated during the surgical proceedings near the fenestration.

The fenestration may be provided with a separate absorbent structure encircling the fenestration, such as an O-shaped sponge. The fenestration may furthermore be provided with a pouch or tunnel, permitting passage of the newborn infant therethrough. The pouch or tunnel may be in the form of a transparent and flexible structure.

Optionally, the surgical drape comprises an absorbent pad arranged proximal to the fenestration. Optionally, the absorbent pad is a hydrophilic nonwoven pad, such as a hydrophilic viscose nonwoven pad. The absorbent pad may be arranged below, as seen in the longitudinal direction, and adjacent the fenestration or below and adjacent a pouch or tunnel of the fenestration.

Optionally, the first and second limb portion are arranged at a shortest distance of 10 cm or less from the respective first and second longitudinal edge of the patient facing sheet. Alternatively, the first and second limb portion may be arranged at a shortest distance of 5 cm or less from the respective first and second longitudinal edge of the patient facing sheet. Optionally, the first and second limb portion are arranged at a shortest distance of 3 cm or less from the respective first and second longitudinal edge of the patient facing sheet. This distance is measured at the base portion of each limb portion, at the point closest to the respective longitudinal edges. Meaning that the distance is measured between the first limb portion and the first longitudinal edge and the second limb portion and the second longitudinal edge. This configuration enhances ease of locating and inserting the patient's arm into the limb portions.

The first and second limb portion may be arranged in the central portion of the upper region.

Optionally, the surgical drape is a caesarean section (C-section) surgical drape.

Optionally, the upper region of the surgical drape has a greater width than the lower region, preferably the width of the upper region is at least 20% greater than the width of the lower region. The upper portion may correspond to an anaesthesia screen to provide a barrier or screen to create a sterile field around the abdomen during a C-section and may require a greater width than needed in the lower portion which may cover the abdomen and the lower part of the patient body during the surgical procedure.

Optionally, wherein a length of the upper region of the surgical drape is from 30% to 50% of the total length of the surgical drape and the length of the lower region of the surgical drape is from 70% to 50% of the total length of the surgical drape.

According to a second aspect, the present disclosure relates to a surgical drape having a lateral extension and a longitudinal extension, the surgical drape being divided in an upper region for arrangement proximal a head of a patient during surgical procedure and a lower region arranged proximal a surgical site of the patient, the upper region being divided in a first lateral section, a central section and a second lateral section, the surgical drape further comprising:
- a surgeon facing barrier sheet being essentially liquid impermeable and transparent, the surgeon facing barrier sheet extending over the first lateral section, the central section and the second lateral section of the upper region,
- a patient facing sheet being essentially opaque, the patent facing sheet extending over the central section of the upper region,
- a fenestration arranged in the lower region, the fenestration permitting access to the surgical site when the surgical drape covers a patient during surgical procedures,
- a first and a second limb portion arranged in the surgeon facing barrier sheet in the upper region of the surgical drape, the first and second limb portions each comprises a base portion attached to the surgical drape, a closed end portion and a sleeve portion extending between the base portion and the end portion,
- the upper region of the surgical drape having a greater width than the lower region, preferably the width of the upper region is at least 20% greater than the width of the lower region,
- the surgeon facing barrier sheet extends beyond the patient facing sheet along a respective first and second longitudinal edge of the patient facing sheet in the upper region, and
wherein the surgical drape has an unfolded configuration and a folded configuration, wherein:
in the unfolded configuration,
   - the upper region comprises an upper region first longitudinal side edge and an upper region second longitudinal side edge, each extending in the longitudinal direction,
   - the lower region comprises a lower region first longitudinal side edge and a lower region second longitudinal side edge, each extending in the longitudinal direction,
in the folded configuration
   - the first lateral section of the upper region is folded along a first folding line extending in the longitudinal direction to form a first folded stack wherein the first folded stack is defined by a first stack outer longitudinal stack edge and a first stack inner longitudinal stack edge, wherein the first lateral section is folded such that:
      ∘ the first stack outer longitudinal stack edge is substantially aligned with the lower region first longitudinal side edge,
   - the second lateral section of the upper region is folded along a second folding line extending in the longitudinal direction to form a second folded stack wherein the second folded stack is defined by a second stack outer longitudinal stack edge and a second stack inner longitudinal stack edge, wherein the second lateral section is folded such that:
      ∘ the second stack outer longitudinal stack edge is coinciding with the lower region second longitudinal side edge,
   - the surgical drape is folded along a third folding line extending in the longitudinal direction, from the upper region to the lower region, to form a third folded stack wherein the third folded stack is defined by a third stack outer longitudinal stack edge and a third stack inner longitudinal stack edge, wherein the third stack is folded such that:
      ∘ the first stack inner longitudinal stack edge is aligned with the third stack inner longitudinal stack edge and the first stack outer longitudinal stack edge is aligned with the third stack outer longitudinal stack edge,
   - the surgical drape is folded along a fourth folding line extending in the longitudinal direction, from the upper region to the lower region, to form a fourth folded stack wherein the fourth folded stack is defined by a fourth stack outer longitudinal stack edge and a fourth stack inner longitudinal stack edge, wherein the fourth stack is folded such that:
      ∘ the second stack inner longitudinal stack edge is aligned with the fourth stack inner longitudinal stack edge and the second stack outer longitudinal stack edge is aligned with the fourth stack outer longitudinal stack edge.

The folding of the surgical drape according to the second aspect, offers a particularly advantageous and straightforward approach. The method allows the surgical drape to be positioned over the patient, along the longitudinal extension, and then unfolded in a single step in each direction, as seen in the lateral extension. By gripping the longitudinal edges of the surgical drape on each side and pulling laterally, the entire surgical drape unfolds smoothly and efficiently covering the patient with ease.

Optionally, the first lateral section is folded such that the upper region first longitudinal side edge is coinciding with, or at least directed towards, the outer longitudinal first stack edge. This further facilitates the unfolding since the edges are directed outwardly and may be pulled directly outwardly upon unfolding of the drape. This also means that the upper region first longitudinal side edge may be aligned with the outer longitudinal first stack edge or positioned inwardly, such as slightly inwards, from it. This alignment or inward positioning helps to prevent direct contact direct between the patient and the upper region first longitudinal side edge when folded drape is placed on the patient, thereby maintaining a sterile environment on the surgeon facing side of the surgical drape.

The first folding line may be aligned with, or extend outwardly of, the first longitudinal edge of the patient facing sheet in the upper region. This may facilitate folding, as the material is thinner at the folding line, and folding lines on the patient facing sheet is avoided.

Optionally, the second lateral section is folded such the upper region second longitudinal side edge is coinciding with, or at least directed towards, the outer longitudinal second stack edge. This also means that the upper region second longitudinal side edge may be aligned with the outer longitudinal second stack edge or positioned inwardly, such as slightly inwards, from it.

The second folding line may be aligned with, or extend outwardly of, the second longitudinal edge of the patient facing sheet in the upper region.

Optionally, the first and/or second stack are folded in a Z-fold or C-fold configuration.

Optionally, the third and/or fourth stack are folded in a Z-fold or C-fold configuration.

Optionally, the surgical drape is further folded along a fifth folding line extending in the lateral direction and in the upper region to form a fifth folded stack.

Optionally, the surgical drape is further folded along a sixth folding line extending in the lateral direction and in the lower region to form a sixth folded stack.

Optionally, the surgical drape is folded along a plurality of folding lines extending in the lateral direction to form one folded stack or two folded stacks.

Optionally, the surgical drape is a surgical drape according to the first aspect.

### BRIEF DESCRIPTION OF THE DRAWINGS

Aspects and embodiments are now described, by way of example, with reference to the accompanying drawings, in which:
Fig 1 shows a plan view of a surgical drape according to the present disclosure from a surgeon facing side.
Fig 2 shows a plan view of the surgical shown in Fig. 1 from a patent facing side.
Figs. 3a-3b show a folded surgical drape according to the present disclosure.

### DETAILED DESCRIPTION

The aspects of the present disclosure will now be described more fully hereinafter with reference to the accompanying drawings, in which certain embodiments of the invention are shown.

These aspects may, however, be embodied in many different forms and should not be construed as limiting; rather, these embodiments are provided by way of example so that this disclosure will be thorough and complete, and to fully convey the scope of all aspects of invention to those skilled in the art. Like numbers refer to like elements throughout the description.

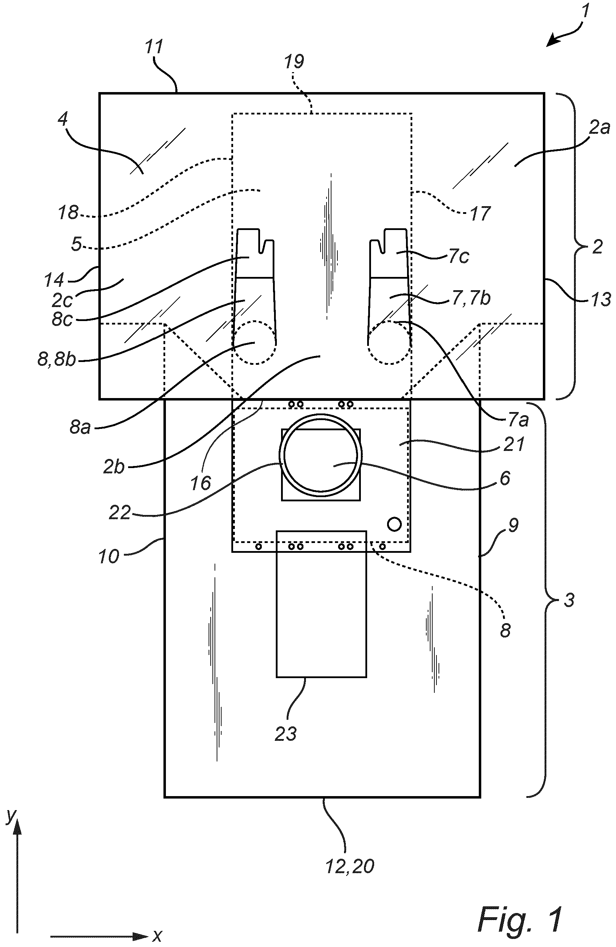

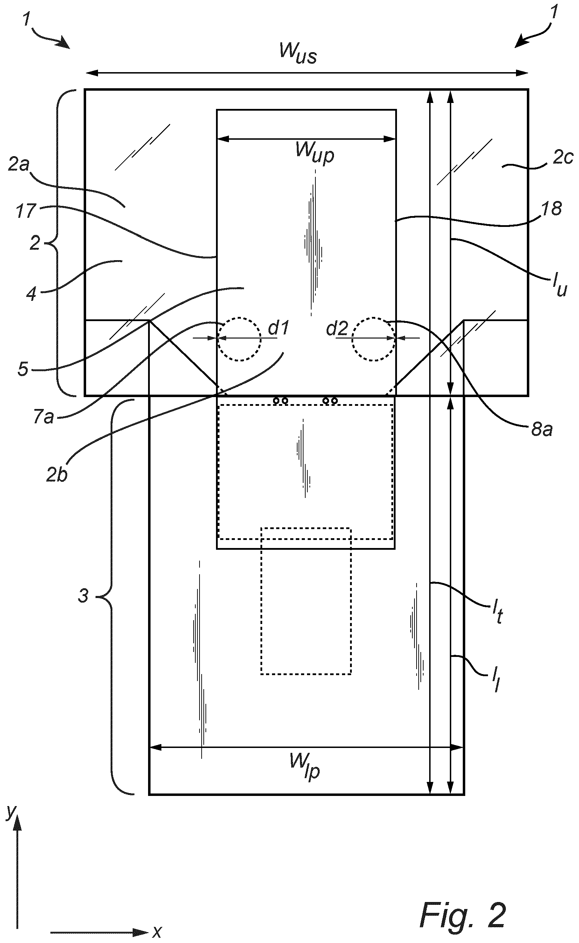

Figs. 1 and 2 illustrate a surgical drape 1 according to the present disclosure. The surgical drape 1 in Figs 1 and 2 is a C-section surgical drape. Fig. 1 shows a view from the surgeon facing side and Fig. 2 is a view from the patient facing side of the surgical drape 1.

The surgical drape 1 has a lateral extension x and a longitudinal extension y. The surgical drape 1 is divided in an upper region 2 for arrangement proximal a head of a patient during surgical procedure and a lower region 3 arranged proximal a surgical site of the patient during surgical procedure. The upper region 2 is divided in a first lateral section 2a, a central section 2b and a second lateral section 2c.

A shown in Fig. 2. a length lᵤ of the upper region of the surgical drape may be from 30% to 50% of the total length lt of the surgical drape and the length lₗ of the lower region of the surgical drape may be from 70% to 50% of the total length lt of the surgical drape.

The surgical drape 1 further comprises a surgeon facing barrier sheet 4. The surgeon facing barrier sheet 4 is essentially liquid impermeable and transparent. The surgeon facing barrier sheet 4 may for example be a clear plastic liquid impervious polyethylene film, such as a non-breathable polyethylene film with a basis weight within the range of from 20 g/m² - 50 g/m², preferably within the range of from 22 g/m² -40 g/m². Optionally, the surgeon facing barrier sheet has a liquid penetration of ≥ 100 cm H₂O, as measured according to ISO 811.

The surgeon facing barrier sheet 4 extends over the first lateral section 2a, the central section 2b and the second lateral section 2c of the upper region 2.

The surgical drape 1 further comprises a patient facing sheet 5 being essentially opaque. The patent facing sheet 5 extends over the central section 2b of the upper region 2.

A fenestration 6 is positioned in the lower region 3, allowing access to the surgical site when the surgical drape 1 is placed over a patient during surgical procedures. In the embodiment shown in Fig. 1, the fenestration is equipped with a pouch or tunnel 21, designed for the passage of the newborn infant. This pouch or tunnel may be in the form of a transparent and flexible structure. To manage fluids generated near the fenestration during the surgical proceedings, an absorbent structure 22, such as a sponge, surrounds the fenestration 6.

An absorbent pad 23 is furthermore arranged in the lower region 3, below and adjacent the fenestration 6 or below and adjacent the pouch/tunnel 22. The absorbent pad 23 may be a hydrophilic nonwoven pad, such as a hydrophilic viscose nonwoven pad.

The surgical drape is outlined by a first and a second longitudinal edge 9,10, an upper edge 11 and a lower edge 12. As illustrated in the figures, the drape 1 has a T-shaped design, with the upper region 2 of the surgical drape 1 being wider than the lower region, preferably the width of the upper region 2 is at least 20% greater than the width of the lower region 3.

The surgeon facing barrier sheet 4 is outlined by a first and a second longitudinal edge 13,14, an upper edge 15 and a lower edge 16, the lower edge 16 coinciding with a lower border of the second region 2. The surgeon facing barrier sheet 4 has an upper region width wᵤₛ, as measured in the lateral x direction and in the upper region 2, between the first and second longitudinal edges 13,14.

The patient facing sheet 5 is outlined by a first and a second longitudinal edge 17,18, an upper edge 19 and a lower edge 20. The patient facing sheet 5 has an upper region width wᵤₚ, as measured in the lateral x direction and in the upper region 2, between the first and second longitudinal edges 17,18.

As may be seen in Figs. 1 and 2, the upper region width wᵤₛ of the surgeon facing barrier sheet 4 is greater than the upper region width wᵤₚ of the patient facing sheet 5, such that the surgeon facing barrier sheet 5 extends beyond the patient facing sheet 4 on a respective first and second longitudinal edge of the patient facing sheet.

Preferably, the upper region width wᵤₛ of the surgeon facing barrier sheet 4 is at least 30% greater than upper region width wᵤₚ of the patient facing sheet, preferably at least 50% greater or at least 100% greater.

In the embodiment shown in Figs 1 and 2, the patient facing sheet 5 serves as the main sheet extending across the lower region 3 of the surgical drape 1, such as covering the lower region 3 entirely. In this embodiment, a width wₗₚ of the patient facing sheet 5 in the lower region is greater than the width wᵤₚ of the patient facing sheet 5 in the upper region 2. Preferably the width wₗₚ of the patient facing sheet 5 is at least 30% greater in the lower region 3 than the width wᵤₚ in the upper region 2. Optionally the width wₗₚ of the patient facing sheet 5 in the lower region is within the range of from 30% to 75% greater than width wᵤₚ of the patient facing sheet 5 in the upper region 2. However, in an alternative embodiment, the patient facing sheet 5 is joined to a second patient facing sheet extending over the lower region 3 of the surgical drape, such as along a joint in the area bridging the upper region 2 and the lower region 3.

A first and a second limb portion 7,8 are arranged in the surgeon facing barrier sheet 4 in the upper region 2 of the surgical drape 1. The first and second limb portions 7,8 each comprises a base portion 7a,8a attached to the surgical drape, or more specifically to the surgeon facing barrier sheet 4, a closed end portion 7c,8c and a sleeve portion 7b,8b extending between the base portion 7a,8a and the end portion 7c, 8c.

The first and second limb portions 7,8 are arranged with a shortest distance d₁/d₂ of 10 cm or less from a respective first and second longitudinal edge of the patient facing sheet 17,18. In these figures, the limb portions are 7,8 are aligned edge-to-edge with the respective first and second longitudinal edge, resulting in d₁ and d₂ being close to o cm.

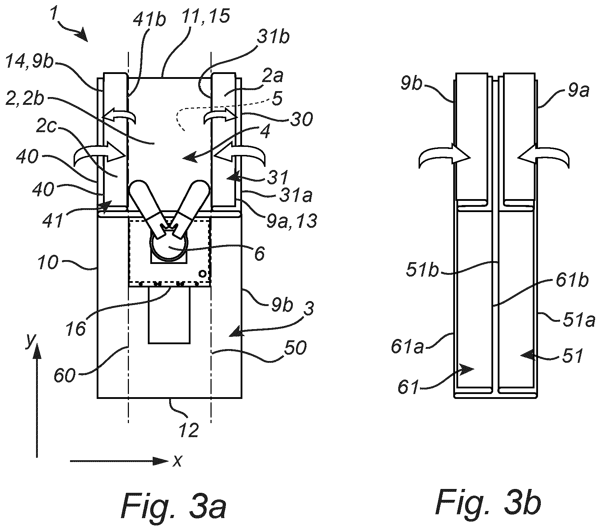

Figure 3 illustrates folding of a surgical drape 1. The surgical drape 1 in Figure 3 is a surgical drape according to Figures 1 and 2. The surgical drape 1 has a lateral extension x and a longitudinal extension y. The surgical drape 1 is divided in an upper region 2 for arrangement proximal a head of a patient during surgical procedure and a lower region 3 arranged proximal a surgical site of the patient during surgical procedure. The upper region 2 is divided in a first lateral section 2a, a central section 2b and a second lateral section 2c.

The surgical drape 1 further comprises a surgeon facing barrier sheet 4. The surgeon facing barrier sheet 4 is essentially liquid impermeable and transparent.

The surgeon facing barrier sheet 4 extends over the first lateral section 2a, the central section 2b and the second lateral section 2c of the upper region 2.

The surgical drape 1 further comprises a patient facing sheet 5 being essentially opaque. The patent facing sheet 5 extends over the central section 2b of the upper region 2.

A fenestration 6 is positioned in the lower region 3, allowing access to the surgical site when the surgical drape 1 is placed over a patient during surgical procedures.

A first and a second limb portion 7,8 are arranged in the surgeon facing barrier sheet 4 in the upper region 2 of the surgical drape 1. The first and second limb portions 7,8 each comprises a base portion 7a,8a attached to the surgical drape, or more specifically to the surgeon facing barrier sheet 4, a closed end portion 7c,8c and a sleeve portion 7b,8b extending between the base portion 7a,8a and the end portion 7c,8c.

The surgical drape is outlined by a first and a second longitudinal edge 9,10, an upper edge 11 and a lower edge 12. As illustrated in the figures, the drape 1 has a T-shaped design, with the upper region 2 of the surgical drape 1 being wider than the lower region, preferably the width of the upper region 2 is at least 20% greater than the width of the lower region 3.

The surgeon facing barrier sheet 4 is outlined by a first and a second longitudinal edge 13,14, an upper edge 15 and a lower edge 16. The surgeon facing barrier sheet 4 has an upper region width, as measured in the lateral x direction and in the upper region 2, between the first and second longitudinal edges 13,14.

The patient facing sheet 5 is outlined by a first and a second longitudinal edge 17,18, an upper edge 19 and a lower edge 20. The patient facing sheet 5 has an upper region width, as measured in the lateral x direction and in the upper region 2, between the first and second longitudinal edges 17,18.

As illustrated in Fig. 3a, the upper region 2 comprises an upper region first longitudinal side edge 9a and an upper region second longitudinal side edge 10a, each extending in the longitudinal y direction.

The lower region 3 comprises a lower region first longitudinal side edge 9b and a lower region second longitudinal side edge 10b, each extending in the longitudinal y direction.

In the folded configuration, the first lateral section 2a of the upper region 2 is folded along a first folding line 30 extending in the longitudinal y direction to form a first folded stack 31 and wherein the first folded stack 31 is defined by a first stack outer longitudinal stack edge 31a and a first stack inner longitudinal stack edge 31b.

The first lateral section is folded such that the first stack outer longitudinal stack edge 31a is substantially aligned with the lower region first longitudinal side edge 9b.

The second lateral section 2c of the upper region 2 is folded along a second folding line 40 extending in the longitudinal y direction to form a second folded stack 41 wherein the second folded stack 41 is defined by a second stack outer longitudinal stack edge 41a and a second stack inner longitudinal stack edge 41b.

The second lateral section 2c is folded such that the second stack outer longitudinal stack edge 41a is coinciding with the lower region second longitudinal side edge.

The surgical drape 1 is furthermore folded along a third folding line 50 extending in the longitudinal y direction, from the upper region 2 to the lower region 3, to form a third folded stack 51.

The third folded stack 51 is defined by a third stack outer longitudinal stack edge 51a and a third stack inner longitudinal stack edge 51b, wherein the third stack 51 is folded such that the first stack inner longitudinal stack edge 31b is aligned with the third stack inner longitudinal stack edge 51b and the first stack outer longitudinal stack edge 31a is aligned with the third stack outer longitudinal stack edge 51a.

The surgical drape is folded along a fourth folding line 60 extending in the longitudinal y direction, from the upper region 2 to the lower region 3, to form a fourth folded stack 61 wherein the fourth folded stack 61 is defined by a fourth stack outer longitudinal stack edge 61a and a fourth stack inner longitudinal stack edge 61b.

The fourth stack 61 is folded such that the second stack inner longitudinal stack edge 41b is aligned with the fourth stack inner longitudinal stack edge 61b and the second stack outer longitudinal stack edge 41a is aligned with the fourth stack outer longitudinal stack edge 61a.

The first lateral section 2a in Figs. 3a and 3b is furthermore folded such that the upper region first longitudinal side edge 9a is coinciding with, or at least directed towards, the outer longitudinal first stack edge 31a.

The second lateral section 2c is folded such that the upper region second longitudinal side edge 10a is coinciding with, or at least directed towards, the outer longitudinal second stack edge 41a.

The first and/or second stack 31,41 may optionally be folded in a Z-fold or C-fold configuration.

The third and/or fourth stack 51,61 may optionally folded in a Z-fold or C-fold configuration.

The aspects of the present disclosure have mainly been described above with reference to a few embodiments and examples thereof. However, as is readily appreciated by a person skilled in the art, other embodiments than the ones disclosed above are equally possible within the scope of the invention, as defined by the appended patent claims.

Thus, while various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

## Claims

1. A surgical drape (1) having a lateral extension (x) and a longitudinal extension (y), the surgical drape being divided in an upper region (2) for arrangement proximal a head of a patient during surgical procedure and a lower region (3) arranged proximal a surgical site of the patient, the upper region being divided in a first lateral section (2a), a central section (2b) and a second lateral section (2c), the surgical drape further comprising:
- a surgeon facing barrier sheet (4) being essentially liquid impermeable and transparent, the surgeon facing barrier sheet extending over the first lateral section, the central section and the second lateral section of the upper region,
- a patient facing sheet (5) being essentially opaque, the patent facing sheet extending over the central section of the upper region,
- a fenestration (6) arranged in the lower region, the fenestration permitting access to the surgical site when the surgical drape covers a patient during surgical procedures,
- a first and a second limb portion (7,8) arranged in the surgeon facing barrier sheet in the upper region of the surgical drape, the first and second limb portions each comprises a base portion (7a,8a) attached to the surgical drape, a closed end portion (7c,8c) and a sleeve portion (7b,8b) extending between the base portion and the end portion,
wherein the surgeon facing barrier sheet extends beyond the patient facing sheet along a respective first and second longitudinal edge of the patient facing sheet in the upper region.

2. The surgical drape according to claim 1, wherein the surgeon facing barrier sheet has a upper region width (wᵤₛ) and wherein the patient facing sheet has a upper region width (wₚ), as measured in the lateral (x) direction and in the upper region, and wherein upper region width (wᵤₛ) of the surgeon facing barrier sheet is at least 30% greater than upper region width (wᵤₚ) of the patient facing sheet, preferably at least 50% greater or at least 100% greater.

3. The surgical drape according to claim 1 or 2, wherein the patient facing sheet is an absorbent sheet.

4. The surgical drape according to any one of the preceding claims, wherein the patient facing sheet is a main sheet further extending over the lower region of the surgical drape.

5. The surgical drape according to claim 4, wherein a width (wₗₚ) of the patient facing sheet in the lower region is greater than a width (wᵤₚ) of the patient facing sheet in the upper region, preferably the width (wₗₚ) of the patient facing sheet in the lower region is at least 30% greater than the width (wᵤₚ) in the upper region, optionally the width (wₗₚ) of the patient facing sheet is within the range of from 30% to 75% greater in the lower region than the width (wᵤₚ) in the upper region.

6. The surgical drape according to any one of the preceding claims, wherein the first and second limb portion are arranged with a shortest distance (d₁,d₂) of 10 cm or less from a respective first and second longitudinal edge (17,18) of the patient facing sheet.

7. The surgical drape according to any one of the preceding claims, wherein the surgical drape is a caesarean section (C-section) surgical drape.

8. The surgical drape according to any one of the preceding claims, wherein the upper region of the surgical drape has a greater width than the lower region, preferably the width of the upper region is at least 20% greater than the width of the lower region.

9. The surgical drape according to any one of the preceding claims, wherein a length (lᵤ) of the upper region of the surgical drape is from 30% to 50% of the total length (lₜ) of the surgical drape and the length (li) of the lower region of the surgical drape is from 70% to 50% of the total length (lt) of the surgical drape.

10. A surgical drape (1) having a lateral extension (x) and a longitudinal extension (y), the surgical drape being divided in an upper region (2) for arrangement proximal a head of a patient during surgical procedure and a lower region (3) arranged proximal a surgical site of the patient, the upper region being divided in a first lateral section (2a), a central section (2b) and a second lateral section (2c), the surgical drape further comprising:
- a surgeon facing barrier sheet (4) being essentially liquid impermeable and transparent, the surgeon facing barrier sheet extending over the first lateral section, the central section and the second lateral section of the upper region,
- a patient facing sheet (5) being essentially opaque, the patent facing sheet extending over the central section of the upper region,
- a fenestration (6) arranged in the lower region, the fenestration permitting access to the surgical site when the surgical drape covers a patient during surgical procedures,
- a first and a second limb portion (7,8) arranged in the surgeon facing barrier sheet in the upper region of the surgical drape, the first and second limb portions each comprises a base portion (7a,8a) attached to the surgical drape, a closed end portion (7c,8c)and a sleeve portion (7b,8b)extending between the base portion and the end portion,
- the upper region of the surgical drape having a greater width than the lower region, preferably the width of the upper region is at least 20% greater than the width of the lower region,
- the surgeon facing barrier sheet extends beyond the patient facing sheet along a respective first and second longitudinal edge of the patient facing sheet in the upper region, and
wherein the surgical drape has an unfolded configuration and a folded configuration, wherein;
in the unfolded configuration,
- the upper region comprises an upper region first longitudinal side edge (9a) and an upper region second longitudinal side edge (10a), each extending in the longitudinal (y) direction,
- the lower region comprises a lower region first longitudinal side edge (9b) and a lower region second longitudinal side edge (10b), each extending in the longitudinal (y) direction,
in the folded configuration
- the first lateral section of the upper region is folded along a first folding line (30) extending in the longitudinal (y) direction to form a first folded stack (31) wherein the first folded stack is defined by a first stack outer longitudinal stack edge (31a) and a first stack inner longitudinal stack edge (31b), wherein the first lateral section is folded such that:
∘ the first stack outer longitudinal stack edge is substantially aligned with the lower region first longitudinal side edge,
- the second lateral section of the upper region is folded along a second folding line (40) extending in the longitudinal (y) direction to form a second folded stack (41) wherein the second folded stack is defined by a second stack outer longitudinal stack edge (41a) and a second stack inner longitudinal stack edge (41b), wherein the second lateral section is folded such that:
∘ the second stack outer longitudinal stack edge is coinciding with the lower region second longitudinal side edge,
- the surgical drape is folded along a third folding line (50) extending in the longitudinal (y) direction, from the upper region to the lower region, to form a third folded stack (51) wherein the third folded stack is defined by a third stack outer longitudinal stack edge (51a) and a third stack inner longitudinal stack edge (51b), wherein the third stack is folded such that:
∘ the first stack inner longitudinal stack edge is aligned with the third stack inner longitudinal stack edge and the first stack outer longitudinal stack edge is aligned with the third stack outer longitudinal stack edge,
- the surgical drape is folded along a fourth folding line (60) extending in the longitudinal (y) direction, from the upper region to the lower region, to form a fourth folded stack (61) wherein the fourth folded stack is defined by a fourth stack outer longitudinal stack edge (61a) and a fourth stack inner longitudinal stack edge (61b), wherein the fourth stack is folded such that:
∘ the second stack inner longitudinal stack edge is aligned with the fourth stack inner longitudinal stack edge and the second stack outer longitudinal stack edge is aligned with the fourth stack outer longitudinal stack edge.

11. The surgical drape according to claim 10, wherein the first lateral section is folded such that:
∘ the upper region first longitudinal side edge is coinciding with, or at least directed towards, the outer longitudinal first stack edge.

12. The surgical drape according to claim 10 or 11, wherein the second lateral section is folded such that:
∘ the upper region second longitudinal side edge is coinciding with, or at least directed towards, the outer longitudinal second stack edge.

13. The surgical drape according to any one of claims 10 to 12, wherein the first and/or second stack are folded in a Z-fold or C-fold configuration.

14. The surgical drape according to any one of claims 10 to 13, wherein the third and/or fourth stack are folded in a Z-fold or C-fold configuration.

15. The surgical drape according to any one of claims 10 to 14, wherein the surgical drape is a drape according to any one of claims 1 to 9.
